# EUROPEAN PATENT SPECIFICATION

(11) **EP 2 536 449 B1**
(45) Date of publication and mention of the grant of the patent: **24.09.2014**
(21) Application number: 11705384.3
(22) Date of filing: 14.02.2011
(51) Int. Cl.: A61M 5/178

(54) **SYRINGE PRODUCTS AND RELATED METHODS AND USES**
SPRITZENPRODUKTE, ZUGEHÖRIGE VERFAHREN UND VERWENDUNG
PRODUITS SERINGUES, MÉTHODES ET UTILISATIONS AFFÉRENTES

(30) Priority: 15.02.2010 US 304537 P
(43) Date of publication of application: 26.12.2012
(73) Proprietor: Mallinckrodt LLC, Hazelwood, MO 63042 (US)
(72) Inventor: SCHRADER, Eugene, F., St. Louis, Missouri 63110-3129 (US)
(74) Representative: Chettle, Adrian John
(86) International application number: PCT/US2011/024678
(87) International publication number: WO 2011/100648

(56) References cited:
- WO-A1-2009/041381
- WO-A2-2006/062956
- US-A1- 2008 275 180

## Description

### RELATED APPLICATIONS

This application claims priority to US provisional application serial number 61/304,537 filed on 15 February 2010 and entitled "SYRINGE PRODUCTS AND RELATED METHODS AND USES".

### FIELD OF THE INVENTION

The present invention generally relates to the field of syringes and, more particularly, to the field of syringes having a syringe barrel wall made with polymer compositions.

### BACKGROUND

Various medical procedures require that one or more medical fluids be injected into a patient. For example, medical imaging procedures oftentimes involve the injection of contrast media into a patient, possibly along with saline and/or other fluids. Other medical procedures involve injecting one or more fluids into a patient for therapeutic or palliative purposes. Power injectors may be used for many applications, and particularly those involving a high injection rate or injection at a very controlled rate. With either route, the fluid to be injected is contained within a syringe prior to injection, and more particularly is contained within a syringe barrel of the syringe. During the injection operation, fluid is expelled from the syringe, typically due to fluid pressure created by advancement of a syringe plunger, or piston, within the syringe barrel that causes the fluid to flow out of an exit port located opposite the advancing syringe plunger. With a hand held syringe, the syringe plunger is advanced by pushing with the hand against a pushrod that applies a force to cause the syringe plunger to advance in the syringe barrel.

A power injector generally includes what is commonly referred to as a powerhead. One or more syringes may be mounted to the powerhead in various manners (e.g., detachably; rear-loading; front-loading; side-loading). The syringe plunger is designed to interface with (e.g., contact and/or temporarily interconnect with) an appropriate syringe plunger driver that is incorporated into the powerhead, such that operation of the syringe plunger driver axially advances the associated syringe plunger inside and relative to a barrel of the syringe (i.e., toward or away from the exit port of the syringe). One typical syringe plunger driver is in the form of a ram that is mounted on a threaded lead or drive screw. Rotation of the drive screw in one rotational direction advances the associated ram in one axial direction, while rotation of the drive screw in the opposite rotational direction advances the associated ram in the opposite axial direction.

The walls of the syringe barrel, which contain the fluid within the syringe barrel and with which the fluid is in contact during storage and use, should have certain properties consistent with the needs of the particular application. A syringe barrel wall should have an adequate pressure rating for pressure anticipated within the syringe barrel during an injection operation. A wall should be sufficiently transparent to permit a visual inspection of contents within the syringe barrel, to identify any irregularities of the contents prior to use. A wall should be sufficiently tough that it is not likely to be structurally damaged during storage, transportation, handling or use. A wall should be sufficiently inert with respect to the contents of the syringe with which the wall is in contact. For example, a wall should not contain a significant amount of components that are susceptible to being extracted into the fluid contents of the syringe, which could result in contamination or even chemical alteration of the contents of the syringe. The property of the tendency of components of a wall material to be extractable into fluid contained within the syringe is often referred to as "extractability." A low extractability is generally desirable.

From operational efficiency and quality control perspectives, it would be beneficial to have a single material of construction that could be used for fabrication of syringes designed for a variety of different applications and for use in a variety of different jurisdictions. As will be appreciated, however, different syringe applications will have different requirements, especially with respect to pressure rating for high-pressure applications relative to low-pressure applications. For example, some power injection applications may require syringe barrels having very high pressure ratings, whereas many hand syringe applications may not require such high pressure ratings. Also, different jurisdictions impose different regulatory requirements, even in relation to the same type of syringe application.

Glass compositions have long been used as materials of construction for syringe barrels, and have properties that are adequate for many different applications, due to relatively high mechanical properties, high transparency and low extractability. Glass, however, is very expensive.

Polymer-based compositions have also been used as materials of construction for syringe barrels. However, providing a combination of high pressure rating, transparency, toughness and low extractability has generally led to the design and use of more expensive materials of construction to accommodate high-pressure applications than what may be needed for low-pressure applications. The differences in mechanical requirements between high-pressure and low-pressure applications, together with differences in regulatory requirements in different jurisdictions, has generally been accommodated either by using different materials of construction for the different applications and different jurisdictions or by using a uniform material of construction across different applications and jurisdictions, but with the uniform material being significantly more costly than necessary relative to the requirements for some of the applications or in some of the jurisdictions.

For example, compositions based.on polyolefin polymers, and particularly based on polypropylene polymers, have been used to make syringe barrels by injection molding. Polypropylene polymers that have been used include homopolymers, which contain only propylene repeating units, and copolymers in which the propylene repeating units predominate but in which there is also a small percentage of another repeating unit, often an ethylene repeating unit. To obtain good transparency in such compositions, it is often necessary to add a clarifier to the composition. However adding a clarifier may impair one or more mechanical properties and extractability of the composition. Although there has been much success in using polypropylene compositions for syringe bodies, attaining a good balance between transparency, mechanical properties and extractability has been difficult, especially with respect to an economical composition that is suitable for use across different applications and jurisdictions.

More recently, cyclic olefin copolymer (COC) has been used to make syringe barrels by injection molding. COC is a copolymer between cyclic olefins and linear olefins. COC has been available under the Topas® brand of Topas Advanced Polymers, Inc. in the USA and Topas Advanced Polymers GmbH in Germany. COC exhibits a combination of good transparency, good mechanical properties and low extractability that makes it a material of construction with wide application across a number of different syringe applications and different jurisdictions. COC is, however, very expensive.

EP-A-2196483 discloses a syringe product from which may be derived the characterizing portion of Claim 1 appended hereto.

US-A-2008/275180 discloses a polymer material with an isostatic and syndiotactic component which may be either a metallocene or Ziegler-Natta polymer, and may be a homopolyer or a co-polymer.

### SUMMARY

A first aspect of the present invention is provided by a syringe product as defined in Claim 1 appended hereto. The invention further defines a method according to claim 15 for preparing a syringe and a method according to claim 17 for manufacturing a syringe barrel. The polymer composition may be made into syringe barrels having a combination of transparency, mechanical properties and low extractability that is suitable for use in both high-pressure and low-pressure applications, and with a cost significantly lower than cyclic olefin copolymer.

As used herein, a "metallocene polypropylene polymer" is a polymer in which the propylene repeating unit predominates (at least a majority by weight of propylene repeating units) and which is made using a metallocene catalyst, optionally along with using one or more other catalysts. Metallocene catalysts are based on metallocene compounds. Metallocene polypropylene, sometimes abbreviated as mPP, is distinguished from polypropylene polymer made not using a metallocene catalyst, such as some conventional polypropylenes made using a Ziegler-Natta catalyst not in the presence of a metallocene catalyst. Metallocene catalysts and metallocene polypropylene polymers are known in the art of polyolefin polymer manufacture. For example, reference is made to U.S. Patent Nos. 6,376,407 and 6,518,377 concerning some metallocene polypropylenes and metallocene catalysts. Examples of some commercially-available metallocene polypropylene polymers include Achieve^{™} 1605 and Achieve^{™} 3854 of ExxonMobil Chemical, Metocene HM560R of Lyondell Basell, and M3766 of Total Petrochemicals.

The metallocene polypropylene may have properties suitable for injection molding. In one preferred implementation, the metallocene polypropylene may have a melt flow rate in a range suitable for injection molding. The melt flow rate may be determined, for example, according to ASTM D 1238 or ISO 1133.

The clarifier may be any organophosphate clarifier. The organophosphate may be an organophosphoric ester or a metal salt thereof. For instance, the organophosphate may be a basic polyvalent metal salt of a cyclic organophosphoric ester. Some non-limiting examples of organophosphate clarifiers are provided in U.S. Patent Nos. 4,463,113 and 5,342,868. Preferred organophosphate clarifiers are NA-11 and NA-21 from Amfine Chemical Corporation, with NA-21 being more preferred.

The polymer composition may comprise the organophosphate clarifier in any suitable concentration. The concentration of the organophosphate clarifier should be sufficiently large to provide a desired level of clarification to the polymer composition. The concentration of the organophosphate clarifier should also be sufficiently small so as not to introduce undesirable levels of extractables into the polymer composition. Preferably, the polymer composition is comprised at least 0.1 weight percent and still more preferably at least 0.15 weight percent of the organophosphate clarifier. The polymer composition preferably is comprised of no more than 0.4 weight percent, more preferably no more than 0.3 weight percent, even more preferably no more than 0.25 weight percent and still more preferably no more than 0.20 weight percent of the organophosphate clarifier. One preferred implementation is for the polymer composition to comprise at least 99 weight percent of the metallocene polypropylene polymer and from 0.1 weight percent to 0.25 weight percent of the organophosphate clarifier. The polymer composition may comprise minor amounts of other additives, such as processing aids.

A number of feature refinements and additional features are applicable to the first aspect of the present invention. These feature refinements and additional features may be used individually or in any combination. As such, each of the following features that will be discussed may be, but are not required to be, used with any other feature or combination of features of the first aspect. The following discussion is applicable to the first aspect, up to the start of the discussion of a second aspect of the present invention.

The fluid-containment wall of the syringe barrel may be made entirely or partially of the polymer composition. In one preferred implementation, substantially all of the portion of the syringe barrel through which the plunger is movable may be made of the polymer composition. In another preferred implementation, the syringe barrel may be made substantially entirely of the polymer composition, such as for example through injection molding manufacture of the syringe barrel as a unitary piece.

The syringe may have an actuation mechanism that is hand manipulable or machine manipulable to cause movement of the plunger in the internal volume. In one implementation, the syringe product may be in the form of a hand held syringe. In another implementation, the syringe product may be in the form of a power injector syringe for use with a power injector (e.g., the OptiVantage^{™} DH injection system of Covidien, The Stellent CT injection system of Medrad, The Dual Shot Alpha injection system of Nemato).

The fluid-containment wall may have a transparency of at least 45%, preferably at least 48%, and more preferably at least 49%. In one preferred implementation, the fluid-containment wall may have a transparency of at least 50%. For example, transparency may be determined as specified in the Japanese pharmacopeia.

The fluid-containment wall may have an impact resistance of at least 01 foot pound-force (0.0138 kg-force meter), and preferably at least 0.2 foot pound-force (0.0277 kg-force meter).

The syringe barrel may have a resistance to failure by burst from internal pressure of at least 200 psi (1.38 MPa), or at least 250 psi (1.72 MPa), or at least 300 psi (2.07 MPa) or even at least 350 psi 2.41. In one preferred embodiment the syringe barrel may have a resistance to failure by burst from internal pressure of at least 370 psi. Unless otherwise indicated, pressures are gauge pressures, and not absolute pressures.

The syringe barrel may have a low extractability indicated by extraction test absorbance for an ultraviolet wavelength spectrum of 220 to 240 nanometers of not greater than 0.08 and absorbance for an ultraviolet wavelength spectrum of 241 to 350 nanometers of not greater than 0.05. For example, extractability may be determined as specified in the Japanese pharmacopeia.

The fluid-containment wall of the syringe barrel may have a tubular section with a cylindrical internal volume in which the plunger is moveably disposed.

The syringe product may comprise an injectable medical formulation disposed within the internal volume, such as for administration of the injectable medical formulation to a patient. The injectable medical formulation may be of any composition and for any medical-related use, including for therapeutic, palliative, diagnostic, drug delivery or treatment purposes. By "injectable" it is meant that a formulation is sufficiently flowable to be expelled from a syringe due to the advancement of the plunger in the syringe barrel. The injectable medical formulation will often be in the form of a liquid or in the form of a dispersion with sufficient liquid to impart flowability. The liquid component of the injectable medical formulation may often be an aqueous liquid. Examples of some injectable medical formulations that may be disposed of the internal volume include x-ray contrast media, magnetic resonance contrast media, ultrasound imaging agents, saline, heparin, and analgesics. The syringe may be a prefilled syringe comprising a volume of an injectable medical formulation sealed within the internal volume. Prior to use, the seal may be broken to permit the injectable medical formulation to be expelled from the syringe barrel during use to administer the injectable medical formulation to a patient. In this sense, the seal may be considered to be a temporary seal designed to last only until broken to permit use of the syringe product.

The syringe barrel may comprise a fluid exit port opposite the plunger, optionally with an injectable medical formulation disposed between the plunger and the fluid exit port. The fluid exit port may be provided by a bore of restricted diameter through a neck portion located at a distal end of the syringe barrel opposite the direction of advancement of the plunger, and through which fluid exits the syringe barrel for injection delivery. The fluid exit port may be sealed to inhibit the injectable medical formulation from inadvertently exiting the syringe barrel during transportation, storage and handling prior to use. Prior to use of the syringe, the seal may be broken to permit use of the syringe product for administration of the injectable medical formulation to a patient. Again, in this sense the seal may be considered as a temporary seal designed to last only until broken to permit use of the syringe product.

The syringe product may include a hypodermic needle in fluid communication with the internal volume of the syringe barrel. Alternatively, the syringe product may be in the absence of a hypodermic needle, in which case the syringe product may be adapted to engage with a hypodermic needle prior to use, or may be adapted for use without a hypodermic needle.

A second aspect of the present invention is provided by a method for preparing a filled syringe, comprising introducing a volume of an injectable medical formulation into an internal volume of a syringe product. such as the syringe product of the first aspect of the present invention.

A number of feature refinements and additional features are applicable to the second aspect of the present invention. These feature refinements and additional features may be used individually or in any combination. As such, each of the following features that will be discussed may be, but are not required to be, used with any other feature or combination of feature of the second aspect. The following discussion is applicable to the second aspect, up to the start of the discussion of a third aspect of the present invention.

The method may comprise, after introducing a volume of injectable medical formulation into the internal volume of the syringe product, sealing the volume of injectable medical formulation in the internal volume, such as may be the case when the syringe product is a prefilled syringe product, which may be transported and/or stored in a filled state prior to use.

Any of the feature refinements and additional features presented above with respect to the first aspect of the present invention applies equally as refinements and additional features that are applicable to the syringe product of the second aspect of the present invention.

A method for administering an injectable medical formulation to a patient is disclosed, comprising injecting a volume of the injectable medical formulation into the patient from a syringe product, which may be the syringe product of the first aspect of the present invention.

The injecting may be directly into the patient from the syringe product (e.g., when the syringe product is a hypodermic syringe). Alternatively, the injecting may be from the syringe product through intermediate apparatus and into the patient (e.g., when the syringe product is a power injector syringe). For example, during use, the syringe product may be located away from the patient and the injection may be via intermediate injection tubing that provides fluid communication from an outlet of a syringe barrel to the patient or injection apparatus within or proximate to the patient. Prior to the injecting, the injectable medical formulation may be sealed within an internal volume of the syringe product, such as may be the case for a prefilled syringe product. The injecting may be performed after unsealing (breaking the seal of) the volume of the injectable medical formulation.

A third aspect of the present invention is provided by use of a polymer composition comprising a metallocene polypropylene polymer and an organophosphate clarifier. The use may be for a fluid-containment wall of a syringe barrel, or for a syringe barrel including such a fluid-containment wall or for a syringe product including such a syringe barrel.

The number of feature refinements and additional features are applicable to the third aspect of the present invention. These feature refinements and additional features may be used individually or in any combination. As such, each of the following features that will be discussed may be, but are not required to be, used with any other feature or combination of features of the third aspect.

The fluid-containment wall may be in a syringe barrel as described with respect to the first aspect of the present invention, or in a syringe product of the first aspect of the present invention. The features and refinements of the first aspect of the present invention concerning any of the syringe barrel, the fluid-containment wall or the polymer composition of the first aspect of the present invention apply equally to the third aspect of the present invention. The syringe barrel may be part of the syringe product of the first aspect of the present invention, and the feature refinements and additional features of the syringe product of the first aspect apply equally to such a syringe product with respect to the third aspect of the present invention.

A number of feature refinements and additional features are separately applicable to each of above-noted first, second and third aspects of the present invention. These feature refinements and additional features may be used individually or in any combination in relation to each of the above-noted first, second, third and fourth aspects. Any feature of any other various aspects of the present invention that is intended to be limited to a "singular" context or the like will be clearly set forth herein by terms such as "only," "single," "limited to," or the like. Merely introducing a feature in accordance with commonly accepted antecedent basis practice does not limit the corresponding feature to the singular (e.g., indicating that subject matter includes a feature and does not alone mean that the subject matter includes only a single occurrence of the feature). Moreover, any failure to use phrases such as "at least one" also does not limit the corresponding feature to the singular (e.g., indicating that subject matter includes feature and does not mean that the subject matter includes only a single occurrence of such feature. Use of the phrase "at least generally" or the like in relation to a particular feature encompasses the corresponding characteristic and insubstantial variations thereof (e.g., indicating that a syringe barrel is at least generally cylindrical encompasses the syringe barrel being cylindrical). Finally, a reference of a feature in conjunction with the phrase "in one embodiment", "in one configuration", "in one variation", or the like, does not limit the use of the feature to a single embodiment, configuration, variation, etc.

### BRIEF DESCRIPTION OF THE FIGURES

Figure 1 is a perspective view of one embodiment of a syringe product.
Figure 2 is a perspective view of another embodiment of a syringe product.
Figure 3 is a perspective view of another embodiment of a syringe product.
Figure 4 is a perspective view of another embodiment of a syringe product.
Figure 5 is a perspective view of another embodiment of a syringe product.
Figure 6 is a schematic of one embodiment of a power injector.
Figure 7A is a perspective view of one embodiment of a portable stand-mounted, dual-head power injector.
Figure 7B is an enlarged, partially exploded, perspective view of a powerhead used by the power injector of Figure 7A.
Figure 7C is a schematic of one embodiment of a syringe plunger drive assembly used by the power injector of Figure 7A.

### DETAILED DESCRIPTION

The polymer composition comprising a metallocene polypropylene polymer clarified with an organophosphate clarifier may be used in syringe barrels of any design, and any such syringe barrels may be included in any syringe product.

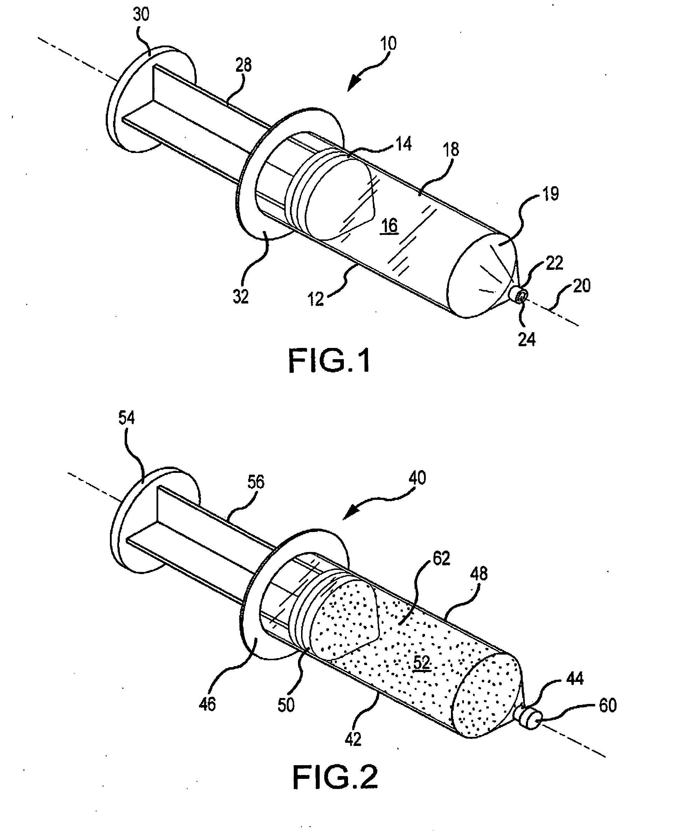

One configuration for a syringe product is illustrated in Figure 1. As shown in Figure 1, a syringe product 10 includes a syringe barrel 12 and a syringe plunger 14 movably disposed within a cylindrically-shaped internal volume 16 of the syringe barrel 12. The syringe barrel 12 includes a fluid-containment wall 18 about the internal volume 16. A front wall portion 19 of the syringe barrel 12 acts as a stop for advancement of the plunger during use. The front wall portion may, for example, be a flat surface or may be tapered to conform with the advancing surface of the plunger 14. At a distal end of the syringe barrel 18, adjacent the front wall portion 19, is a neck 22 with a bore 24 therethrough. The syringe plunger 14 is movable within the internal volume 16 to advance toward or retract away from the neck 22 along a longitudinal axis 20. A syringe actuation member 28 is connected with the plunger 14 and a handle 30. A flange 32 is located at the proximal end of the syringe barrel 12 to facilitate handling of the barrel. Through manipulation of the handle 30 the syringe actuation member 28 may be pushed into or withdrawn from the internal volume 16 to move the plunger 14 in the internal volume 16 toward or away from the neck 22 to create a fluid pressure or fluid suction, respectively, within the internal volume 16. The syringe product 10 is adapted for hand manipulation through grasping the barrel adjacent the flange 32 and pushing or pulling the handle 30 to move the.plunger 14 relative to the syringe barrel 12 within the internal volume 16.

Figure 2 illustrates another configuration of a syringe product in the form of a prefilled syringe. The configuration is similar to the configuration of Figure 1, but prefilled with an injectable medical formulation and capped at the outlet. As shown in Figure 2, a syringe product 40 includes a syringe barrel 42, having a neck 44 at a distal end, a flange 46 at a proximal end, a fluid containment wall 48 and an internal volume 52. A syringe plunger 50 is movably disposed within the internal volume 52 of the syringe barrel 42, and the syringe plunger 50 is movable through manipulation of a handle 54 to push into or withdraw from the internal volume 52 a plunger actuation member 56. The neck 44 has a bore therethrough, which bore is closed at the distal end by a cap 60 over the distal end portion of the neck 44. In the configuration shown in Figure 2, disposed in a portion of the internal volume 52 located between the plunger 50 and the neck 44 is an injectable medical formulation 62, which is sealed within the internal volume between the cap 60 and the plunger 50. To use the syringe product 40 to inject the injectable medical formulation 62 into a patient, the cap 60 may be removed and the bore through the neck 44 may be fluidly connected with a hypodermic needle or injection tubing for delivering at least a portion of the injectable medical formulation 62 to a patient.

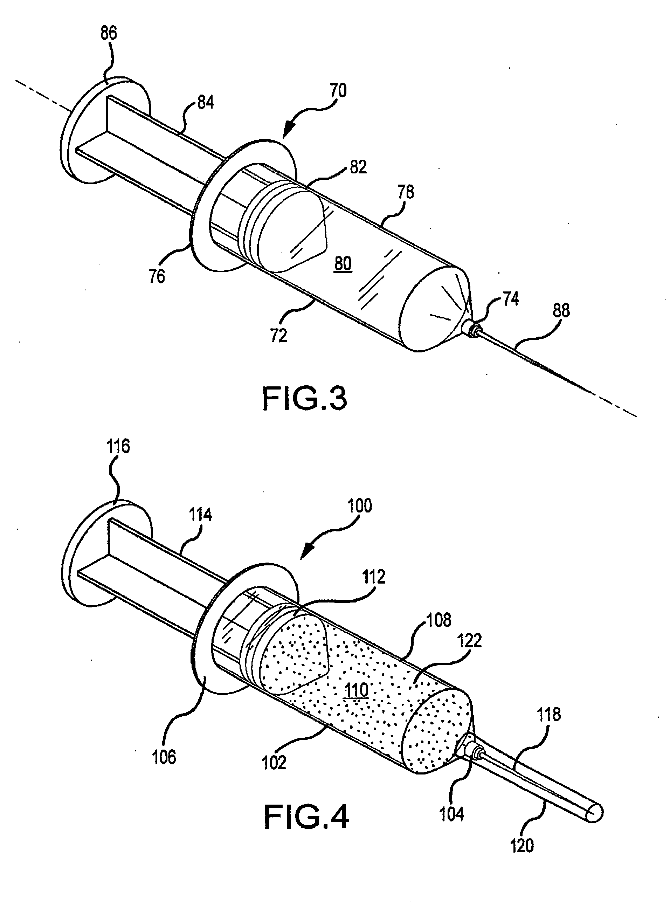

Figure 3 shows another configuration of a syringe product, in the form of a hypodermic syringe. The configuration of Fig. 3 is similar to the configuration of Fig. 1, but with a hypodermic needle. As shown in Figure 3, a syringe product 70 includes a syringe barrel 72 having a neck 74 at a distal end, a flange 76 at a proximal end, a fluid-containment wall 78 and an internal volume 80. Movably disposed within the internal volume 78 is a syringe plunger 82, with the syringe plunger 82 being movable through pushing into or withdrawing from the internal volume 80 a plunger actuation member 84 through manipulation of a handle 86. At the distal end of the barrel 72 is a hypodermic needle 88 containing an internal passage in fluid communication with the internal volume 80, and through which an injectable medical formulation could be injected from the internal volume 78 into a patient by applying pressure to the handle 84 to move the plunger 80 toward the neck 74 to force the injectable medical formulation through a bore through the neck 74 and the internal passage through the needle 88.

Figure 4 illustrates one particular configuration for a syringe product in the form of a prefilled hypodermic syringe. The configuration of Figure 4 is similar to that of Figure 3, but prefilled with an injectable medical formulation and protectively capped over the hypodermic needle. As shown in Figure 4, a syringe product 100 includes a syringe barrel 102 having a neck 104 at a distal end, a flange 106 at a proximal end, a fluid-containment wall 108 and an internal volume 110. A plunger 112 is movably disposed within the internal volume 110, and the plunger 112 may be moved within internal volume 110 by pushing into or withdrawing from the internal volume 110 a plunger actuation member 114 through manipulation of a handle 116. The syringe product 100 includes a hypodermic needle 118 with an internal passage in fluid communication with the internal volume 110. In the configuration shown in Figure 4, an injectable medical formulation 122 is disposed in a portion of the internal volume 110, and the hypodermic needle 118 and the neck 104 are covered by a protective cap 120. To use the syringe product 100 to inject the injectable medical formulation 122 into a patient, the protective cap 120 may be removed and the bore through the neck 104 to permit access to the hypodermic needle 118 for delivering at least a portion of the injectable medical formulation 122 to a patient via the hypodermic needle 118.

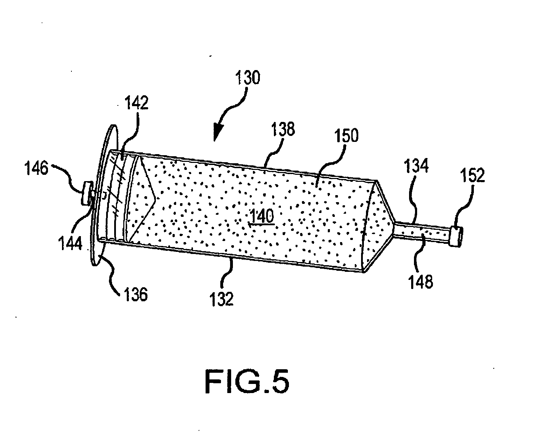

Figure 5 shows one configuration of a syringe product, in the form of a prefilled power injector syringe. As shown in Figure 5, a syringe product 130 has a syringe barrel 132 with a neck 134 located at a distal end, a flange 136 located at a proximal end, a fluid containment wall 138 and an internal volume 140. Disposed within the internal volume 140 is a plunger 142, to which is connected a plunger coupler including a shaft 144 and a head 146, adapted for interfacing with, (e.g., engaging) a power injector syringe plunger driver. A bore 148 though the neck 134 provides a fluid exit port for an injectable medical formulation 150 to be delivered from the syringe product 130 during use. The bore 148 is closed at the distal end by a cap 152 over the distal end of the neck 134. Disposed within a portion of the internal volume 140 is an injectable medical formulation 150 sealed within the syringe barrel between the cap 152 and the plunger 142. To use the syringe product 130 to inject the injectable medical formulation 150 into a patient, the cap 152 may be removed and the plunger coupler may be engaged with a syringe plunger driver of a power injector to advance the syringe plunger 142 into the internal volume to force at least a portion of the injectable medical formulation 150 through the bore 148 and out of the syringe barrel 132, such as via injection tubing which may be fluidly interconnected with the bore 148 to deliver the injectable medical formulation 150 to a patient.

A prefilled syringe (e.g., the syringe product 40 of Figure 2, the syringe product 100 of Figure 4, the syringe product 130 of Figure 5) may be packaged (e.g., enclosed in a box, a plastic container or other packaging structure) separately or as a collection of multiple prefilled syringes packaged as a set. Such packaged prefilled syringe may be distributed by the manufacturer and/or by an intermediate distributor to customers to be temporarily stored ready for use by the customer as needed. Prior to use, the customer may remove a prefilled syringe from the packaging and prepare the syringe for use (e.g., by removing the cap 60 from the syringe product 40 of Figure 2, removing the protective cap 120 from the syringe product 100 of Figure 4, removing the cap 152 from the syringe product 130 of Figure 5).

A method for preparing a filled syringe comprises introducing a volume of an injectable medical formulation into an internal volume of a syringe product, for example, any of the configurations for a syringe product as shown in any of Figures 1-5. In one variation of the method, after introducing a volume of injectable medical formulation into the internal volume of a syringe product, the method may comprise sealing the volume of injectable medical formulation in the internal volume. Such a variation may be used, for example, in the preparation of prefilled syringes.

For example, the syringe product configuration as shown in Figure 1 could be filled with a medical injection formulation within the internal volume of the syringe barrel by retraction of the syringe plunger to create a fluid suction to draw an injectable medical formulation through the bore in the neck into the internal volume of the syringe barrel, after which the bore through the neck could be capped, thereby preparing a prefilled syringe product such as that illustrated in Figure 2, Alternative methods of filling the internal volume could involve introducing an injectable medical formulation into the internal volume through an open proximal end of the syringe barrel prior to inserting the plunger into the internal volume or, with the plunger inserted into the internal volume, introducing the injectable medical formulation into the internal volume through the bore through the neck of the syringe barrel. After filling the internal volume, one variation of the method may comprise fitting the syringe barrel with a hypodermic needle, and may further comprise covering the hypodermic needle, such as with a protective cap, to produce a prefilled syringe product such as that illustrated in Figure 4. Similar procedures may be followed in a method to prepare the prefilled power injector syringe product as shown in Figure 5.

A method for administering an injectable medical formulation to a patient comprises injecting at least a portion of a volume of an injectable medical formulation from an internal volume of a syringe product into the patient. The syringe product may, for example, be of a configuration as shown in any of Figures 1-5. In one variation of the method, the syringe product may initially contain the injectable medical formulation sealed within the internal volume, and the method may comprise, prior to the injecting, unsealing the volume of the injectable medical formulation within the internal volume of the syringe product.

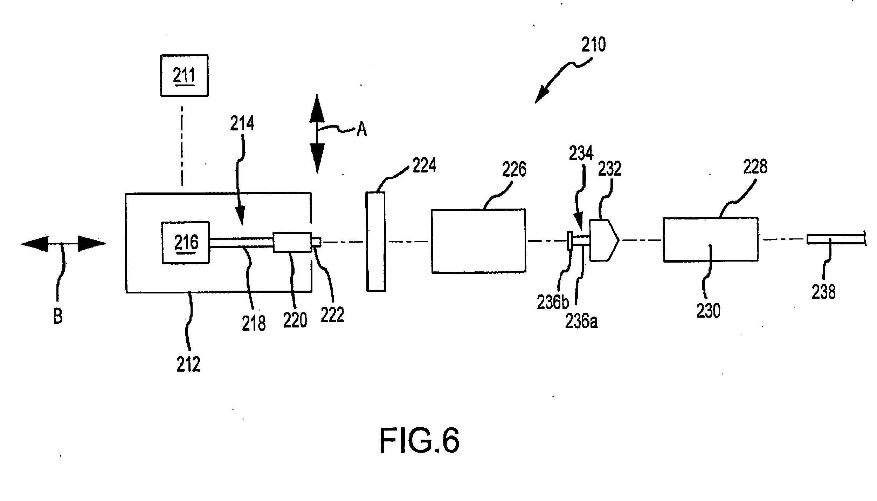

In one variation of a method for administering an injectable medical formulation to a patient, the injecting may be via a power injector. With reference to Figures 6-7C, one configuration for a power injector is described.

Figure 6 presents a schematic of one embodiment of a power injector 210 having a powerhead 212. One or more graphical user interfaces or GUIs 211 may be associated with the powerhead 212. Each GUI 211: 1) may be of any appropriate size, shape, configuration, and/or type; 2) may be operatively interconnected with the powerhead 212 in any appropriate manner; 3) may be disposed at any appropriate location; 4) may be configured to provide any of the following functions: controlling one or more aspects of the operation of the power injector 210; inputting/editing one or more parameters associated with the operation of the power injector 210; and displaying appropriate information (e.g., associated with the operation of the power injector 210); or 5) any combination of the foregoing. Any appropriate number of GUIs 211 may be utilized. In one embodiment, the power injector 210 includes a GUI 211 that is incorporated by a console that is separate from but which communicates with the powerhead 212. In another embodiment, the power injector 210 includes a GUI 211 that is part of the powerhead 212. In yet another embodiment, the power injector 210 utilizes one GUI 211 on a separate console that communicates with the powerhead 212, and also utilizes another GUI 211 that is on the powerhead 212. Each GUI 211 could provide the same functionality or set of functionalities, or the GUIs 211 may differ in at least some respect in relation to their respective functionalities.

A syringe 228 may be installed on the powerhead 212 and, when installed, may be considered to be part of the power injector 210. Some injection procedures may result in a relatively high pressure being generated within the syringe 228. In this regard, it may be desirable to dispose the syringe 228 within a pressure jacket 226. The pressure jacket 226 is typically associated with the powerhead 212 in a manner that allows the syringe 228 to be disposed therein as a part of or after installing the syringe 228 on the powerhead 212. The same pressure jacket 226 will typically remain associated with the powerhead 212, as various syringes 228 are positioned within and removed from the pressure jacket 226 for multiple injection procedures. The power injector 210 may eliminate the pressure jacket 226 if the power injector 210 is configured/utilized for low-pressure injections and/or if the syringe(s) 228 to be utilized with the power injector 210 is (are) of sufficient durability to withstand high-pressure injections without the additional support provided by a pressure jacket 226. In any case, fluid discharged from the syringe 228 may be directed into a conduit 238 of any appropriate size, shape, configuration, and/or type, which may be fluidly interconnected with the syringe 228 in any appropriate manner, and which may direct fluid to any appropriate location (e.g., to a patient).

The powerhead 212 includes a syringe plunger drive assembly or syringe plunger driver 214 that interacts (e.g., interfaces) with the syringe 228 (e.g., a plunger 232 thereof) to discharge fluid from the syringe 228. This syringe plunger drive assembly 214 includes a drive source 216 (e.g., a motor of any appropriate size, shape, configuration, and/or type, optional gearing, and the like) that powers a drive output 218 (e.g., a rotatable drive screw). A ram 220 may be advanced along an appropriate path (e.g., axial) by the drive output 218. The ram 220 may include a coupler 222 for interacting or interfacing with a corresponding portion of the syringe 228 in a manner that will be discussed below.

The syringe 228 includes a plunger or piston 232 that is movably disposed within a syringe barrel 230 (e.g., for axial reciprocation along an axis coinciding with the double-headed arrow B). The plunger 232 may include a coupler 234. This syringe plunger coupler 234 may interact or interface with the ram coupler 222 to allow the syringe plunger drive assembly 214 to retract the syringe plunger 232 within the syringe barrel 230. The syringe plunger coupler 234 may be in the form of a shaft 236a that extends from a body of the syringe plunger 232, together with a head or button 236b. However, the syringe plunger coupler 234 may be of any appropriate size, shape, configuration, and/or type.

Generally, the syringe plunger drive assembly 214 of the power injector 210 may interact with the syringe plunger 232 of the syringe 228 in any appropriate manner (e.g., by mechanical contact; by an appropriate coupling (mechanical or otherwise)) so as to be able to move or advance the syringe plunger 232 (relative to the syringe barrel 230) in at least one direction (e:g., to discharge fluid from the corresponding syringe 228). That is, although the syringe plunger drive assembly 214 may be capable of bi-directional motion (e.g., via operation of the same drive source 216), the power injector 210 may be configured such that the operation of the syringe plunger drive assembly 214 actually only moves each syringe plunger 232 being used by the power injector 210 in only one direction. However, the syringe plunger drive assembly 214 may be configured to interact with each syringe plunger 232 being used by the power injector 210 so as to be able to move each such syringe plunger 232 in each of two different directions (e.g. in different directions along a common axial path).

Retraction of the syringe plunger 232 may be utilized to accommodate a loading of fluid into the syringe barrel 230 for a subsequent injection or discharge, may be utilized to actually draw fluid into the syringe barrel 230 for a subsequent injection or discharge, or for any other appropriate purpose. Certain configurations may not require that the syringe plunger drive assembly 214 be able to retract the syringe plunger 232, in which case the ram coupler 222 and syringe plunger coupler 234 may not be desired. In this case, the syringe plunger drive assembly 214 may be retracted for purposes of executing another fluid delivery operation (e.g., after another prefilled syringe 228 has been installed). Even when a ram coupler 222 and syringe plunger coupler 234 are utilized, these components may or may not be coupled when the ram 220 advances the syringe plunger 232 to discharge fluid from the syringe 228 (e.g., the ram 220 may simply "push on" the syringe plunger coupler 234 or directly on a proximal end of the syringe plunger 232). Any single motion or combination of motions in any appropriate dimension or combination of dimensions may be utilized to dispose the ram coupler 222 and syringe plunger coupler 234 in a coupled state or condition, to dispose the ram coupler 222 and syringe plunger coupler 234 in an un-coupled state or condition, or both.

The syringe 228 may be installed on the powerhead 212 in any appropriate manner. For instance, the syringe 228 could be configured to be installed directly on the powerhead 212. In the illustrated embodiment, a housing 224 is appropriately mounted on the powerhead 212 to provide an interface between the syringe 228 and the powerhead 212. This housing 224 may be in the form of an adapter to which one or more configurations of syringes 228 may be installed, and where at least one configuration for a syringe 228 could be installed directly on the powerhead 212 without using any such adapter. The housing 224 may also be in the form of a faceplate to which one or more configurations of syringes 228 may be installed. In this case, it may be such that a faceplate is required to install a syringe 228 on the powerhead 212 - the syringe 228 could not be installed on the powerhead 212 without the faceplate. When a pressure jacket 226 is being used, it may be installed on the powerhead 212 in the various manners discussed herein in relation to the syringe 228, and the syringe 228 will then thereafter be installed in the pressure jacket 226.

The housing 224 may be mounted on and remain in a fixed position relative to the powerhead 212 when installing a syringe 228. Another option is to movably interconnect the housing 224 and the powerhead 212 to accommodate installing a syringe 228. For instance, the housing 224 may move within a plane that contains the double-headed arrow A to provide one or more of coupled state or condition and an un-coupled state or condition between the ram coupler 222 and the syringe plunger coupler 234.

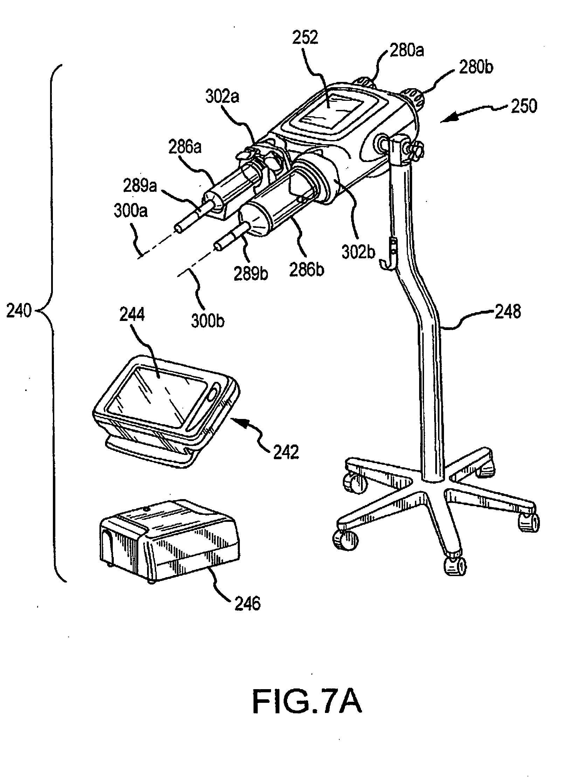

One particular power injector configuration is illustrated in Figure 7A, is identified by a reference numeral 240, and is at least generally in accordance with the power injector 210 of Figure 1. The power injector 240 includes a powerhead 250 that is mounted on a portable stand 248. Two syringes 286a, 286b for the power injector 240 are mounted on the powerhead 250. Fluid may be discharged from the syringes 286a, 286b during operation of the power injector 240.

The portable stand 248 may be of any appropriate size, shape, configuration, and/or type. Wheels, rollers, casters, or the like may be utilized to make the stand 248 portable. The powerhead 250 could be maintained in a fixed position relative to the portable stand 248. However, it may be desirable to allow the position of the powerhead 250 to be adjustable relative to the portable stand 248 in at least some manner. For instance, it may be desirable to have the powerhead 250 in one position relative to the portable stand 248 when loading fluid into one or more of the syringes 286a, 286b, and to have the powerhead 250 in a different position relative to the portable stand 248 for performance of an injection procedure. In this regard, the powerhead 250 may be movably interconnected with the portable stand 248 in any appropriate manner (e.g., such that the powerhead 250 may be pivoted through at least a certain range of motion, and thereafter maintained in the desired position).

It should be appreciated that the powerhead 250 could be supported in any appropriate manner for providing fluid. For instance, instead of being mounted on a portable structure, the powerhead 250 could be interconnected with a support assembly, that in turn is mounted to an appropriate structure (e.g., ceiling, wall, floor). Any support assembly for the powerhead 250 may be positionally adjustable in at least some respect (e.g., by having one or more support sections that may be repositioned relative to one or more other support sections), or may be maintained in a fixed position. Moreover, the powerhead 250 may be integrated with any such support assembly so as to either be maintained in a fixed position or so as to be adjustable relative the support assembly.

The powerhead 250 includes a graphical user interface or GUI 252. This GUI 252 may be configured to provide one or any combination of the following functions: controlling one or more aspects of the operation of the power injector 240; inputting/editing one or more parameters associated with the operation of the power injector 240; and displaying appropriate information (e.g., associated with the operation of the power injector 240). The power injector 240 may also include a console 242 and powerpack 246 that each may be in communication with the powerhead 250 in any appropriate manner (e.g., via one or more cables), that may be placed on a table or mounted on an electronics rack in an examination room or at any other appropriate location, or both. The powerpack 246 may include one or more of the following and in any appropriate combination: a power supply for the injector 240; interface circuitry for providing communication between the console 242 and powerhead 250; circuitry for permitting connection of the power injector 240 to remote units such as remote consoles, remote hand or foot control switches, or other original equipment manufacturer (OEM) remote control connections (e.g., to allow for the operation of power injector 240 to be synchronized with the x-ray exposure of an imaging system); and any other appropriate componentry. The console 242 may include a touch screen display 244, which in turn may provide one or more of the following functions and in any appropriate combination: allowing an operator to remotely control one or more aspects of the operation of the power injector 240; allowing an operator to enter/edit one or more parameters associated with the operation of the power injector 240; allowing an operator to specify and store programs for automated operation of the power injector 240 (which can later be automatically executed by the power injector 240 upon initiation by the operator); and displaying any appropriate information relation to the power injector 240 and including any aspect of its operation.

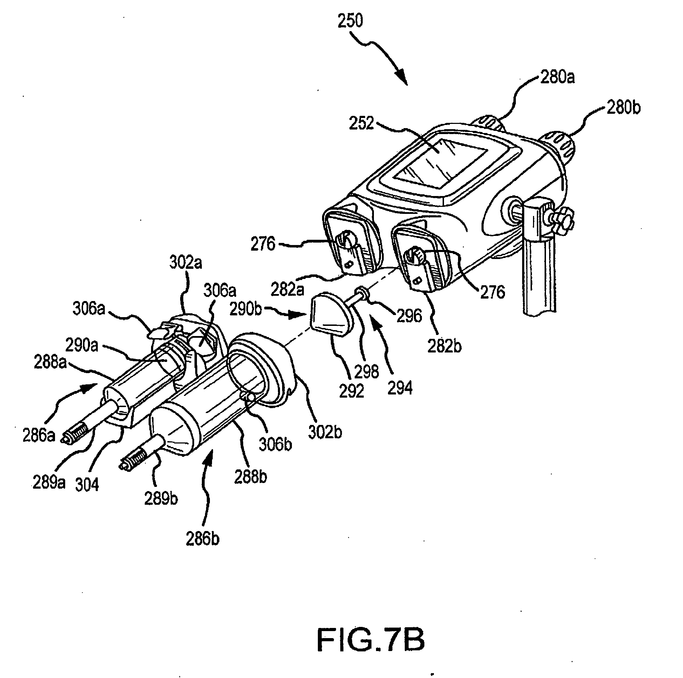

Various details regarding the integration of the syringes 286a, 286b with the powerhead 250 are presented in Figure 7B. Each of the syringes 286a, 286b includes the same general components. The syringe 286a includes plunger or piston 290a that is movably disposed within a syringe barrel 288a. Movement of the plunger 290a along an axis 300a (Figure 7A) via operation of the powerhead 250 will discharge fluid from within a syringe barrel 288a through a nozzle 289a of the syringe 286a. An appropriate conduit (not shown) will typically be fluidly interconnected with the nozzle 289a in any appropriate manner to direct fluid to a desired location (e.g., a patient). Similarly, the syringe 286b includes plunger or piston 290b that is movably disposed within a syringe barrel 288b. Movement of the plunger 290b along an axis 300b (Figure 7A) via operation of the powerhead 250 will discharge fluid from within the syringe barrel 288b through a nozzle 289b of the syringe 286b. An appropriate conduit (not shown) will typically be fluidly interconnected with the nozzle 289b in any appropriate manner to direct fluid to a desired location (e.g., a patient).

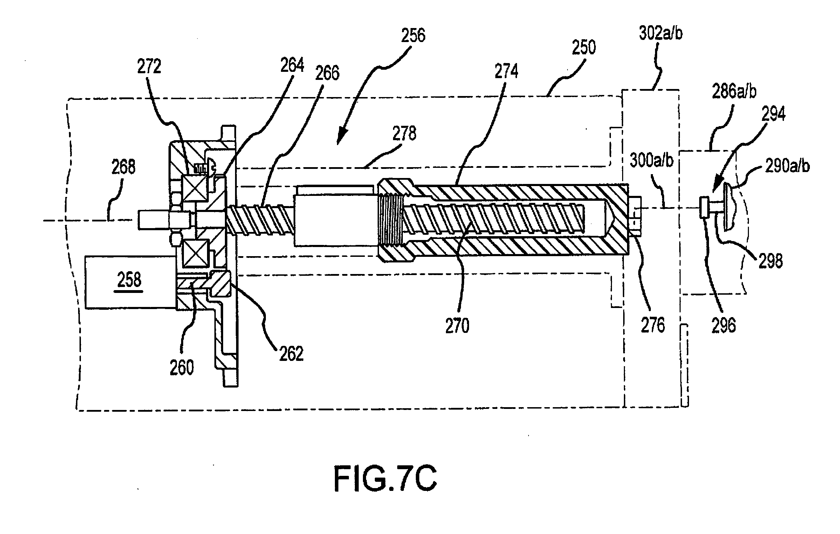

The syringe 286a is interconnected with the powerhead 250 via an intermediate faceplate 302a. This faceplate 302a includes a cradle 304 that supports at least part of the syringe barrel 288a, and which may provide/accommodate any additional functionality or combination of functionalities. A mounting 282a is disposed on and is fixed relative to the powerhead 250 for interfacing with the faceplate 302a. A ram coupler 276 of a ram 274 (Figure 7C), which are each part of a syringe plunger drive assembly or syringe plunger driver 256 (Figure 7C) for the syringe 286a, is positioned in proximity to the faceplate 302a when mounted on the powerhead 250. Details regarding the syringe plunger drive assembly 256 will be discussed in more detail below in relation to Figure 7C. Generally, the ram coupler 276 may be coupled with the syringe plunger 290a of the syringe 286a, and the ram coupler 276 and ram 274 (Figure 7C) may then be moved relative to the powerhead 250 to move the syringe plunger 290a along the axis 300a (Figure 7A). It may be such that the ram coupler 276 is engaged with, but not actually coupled to, the syringe plunger 290a when moving the syringe plunger 290a to discharge fluid through the nozzle 289a of the syringe 286a.

The faceplate 302a may be moved at least generally within a plane that is orthogonal to the axes 300a, 300b (associated with movement of the syringe plungers 290a, 290b, respectively, and illustrated in Figure 7A), both to mount the faceplate 302a on and remove the faceplate 302a from its mounting 282a on the powerhead 250. The faceplate 302a may be used to couple the syringe plunger 290a with its corresponding ram coupler 276 on the powerhead 250. In this regard, the faceplate 302a includes a pair of handles 306a. Generally and with the syringe 286a being initially positioned within the faceplate 302a, the handles 306a may be moved to in turn move/translate the syringe 286a at least generally within a plane that is orthogonal to the axes 300a, 300b (associated with movement of the syringe plungers 290a, 290b, respectively, and illustrated in Figure 7A). Moving the handles 306a to one position moves/translates the syringe 286a (relative to the faceplate 302a) in an at least generally downward direction to couple its syringe plunger 290a with its corresponding ram coupler 276. Moving the handles 306a to another position moves/translates the syringe 286a (relative to the faceplate 302a) in an at least generally upward direction to uncouple its syringe plunger 290a from its corresponding ram coupler 276.

The syringe 286b is interconnected with the powerhead 250 via an intermediate faceplate 302b. A mounting 282b is disposed on and is fixed relative to the powerhead 250 for interfacing with the faceplate 302b. A ram coupler 276 of a ram 274 (Figure 7C), which are each part of a syringe plunger drive assembly 256 for the syringe 286b, is positioned in proximity to the faceplate 302b when mounted to the powerhead 250. Details regarding the syringe plunger drive assembly 256 again will be discussed in more detail below in relation to Figure 7C. Generally, the ram coupler 276 may be coupled with the syringe plunger 290b of the syringe 286b, and the ram coupler 276 and ram 274 (Figure 7C) may be moved relative to the powerhead 250 to move the syringe plunger 290b along the axis 300b (Figure 7A). It may be such that the ram coupler 276 is engaged with, but not actually coupled to, the syringe plunger 290b when moving the syringe plunger 290b to discharge fluid through the nozzle 289b of the syringe 286b.

The faceplate 302b may be moved at least generally within a plane that is orthogonal to the axis 300a, 300b (associated with movement of the syringe plungers 290a, 290b, respectively, and illustrated in Figure 7A), both to mount the faceplate 302b on and remove the faceplate 302b from its mounting 282b on the powerhead 250. The faceplate 302b also may be used to couple the syringe plunger 290b with its corresponding ram coupler 276 on the powerhead 250. In this regard, the faceplate 302b may include a handle 306b. Generally and with the syringe 286b being initially positioned within the faceplate 302b, the syringe 286b may be rotated along its long axis 300b (Figure 7A) and relative to the faceplate 302b. This rotation may be realized by moving the handle 306b, by grasping and turning the syringe 286b, or both, In any case, this rotation moves/translates both the syringe 286b and the faceplate 302b at least generally within a plane that is orthogonal to the axes 300a, 300b (associated with movement of the syringe plungers 290a, 290b, respectively, and illustrated in Figure 7A). Rotating the syringe 286b in one direction moves/translates the syringe 286b and faceplate 302b in an at least generally downward direction to couple the syringe plunger 290b with its corresponding ram coupler 276. Rotating the syringe 286b in the opposite direction moves/translates the syringe 286b and faceplate 302b in an at least generally upward direction to uncouple its syringe plunger 290b from its corresponding ram coupler 276.

As illustrated in Figure 7B, the syringe plunger 290b includes a plunger body 292 and a syringe plunger coupler 294. This syringe plunger coupler 294 includes a shaft 298 that extends from the plunger body 292, along with a head 296 that is spaced from the plunger body 292. Each of the ram couplers 276 includes a larger slot that is positioned behind a smaller slot on the face of the ram coupler 276. The head 296 of the syringe plunger coupler 294 may be positioned within the larger slot of the ram coupler 276, and the shaft 298 of the syringe plunger coupler 294 may extend through the smaller slot on the face of the ram coupler 276 when the syringe plunger 290b and its corresponding ram coupler 276 are in a coupled state or condition. The syringe plunger 290a may include a similar syringe plunger coupler 294 for interfacing with its corresponding ram coupler 276.

The powerhead 250 is utilized to discharge fluid from the syringes 286a, 286b in the case of the power injector 240. That is, the powerhead 250 provides the motive force to discharge fluid from each of the syringes 286a, 286b. One embodiment of what may be characterized as a syringe plunger drive assembly or syringe plunger driver is illustrated in Figure 7C, is identified by reference numeral 256, and may be utilized by the powerhead 250 to discharge fluid from each of the syringes 286a, 286b. A separate syringe plunger drive assembly 256 may be incorporated into the powerhead 250 for each of the syringes 286a, 286b. In this regard and referring back to Figures 7A-B, the powerhead 250 may include hand-operated knobs 280a and 280b for use in separately controlling each of the syringe plunger drive assemblies 256.

Initially and in relation to the syringe plunger drive assembly 256 of Figure 7C, each of its individual components may be of any appropriate size, shape, configuration and/or type. The syringe plunger drive assembly 256 includes a motor 258, which has an output shaft 260. A drive gear 262 is mounted on and rotates with the output shaft 260 of the motor 258. The drive gear 262 is engaged or is at least engageable with a driven gear 264. This driven gear 264 is mounted on and rotates with a drive screw or shaft 266. The axis about which the drive screw 266 rotates is identified by reference numeral 268. One or more bearings 272 appropriately support the drive screw 266.

A carriage or ram 274 is movably mounted on the drive screw 266. Generally, rotation of the drive screw 266 in one direction axially advances the ram 274 along the drive screw 266 (and thereby along axis 268) in the direction of the corresponding syringe 286a, b, while rotation of the drive screw 266 in the opposite direction axially advances the ram 274 along the drive screw 266 (and thereby along axis 268) away from the corresponding syringe 286a/b. In this regard, the perimeter of at least part of the drive screw 266 includes helical threads 270 that interface with at least part of the ram 274. The ram 274 is also movably mounted within an appropriate bushing 278 that does not allow the ram 274 to rotate during a rotation of the drive screw 266. Therefore, the rotation of the drive screw 266 provides for an axial movement of the ram 274 in a direction determined by the rotational direction of the drive screw 266.

The ram 274 includes a coupler 276 that that may be detachably coupled with a syringe plunger coupler 294 of the syringe plunger 290a/b of the corresponding syringe 286a/b. When the ram coupler 276 and syringe plunger coupler 294 are appropriately coupled, the syringe plunger 290a/b moves along with ram 274. Figure 7C illustrates a configuration where the syringe 286a/b may be moved along its corresponding axis 300a/b without being coupled to the ram 274. When the syringe 286a/b is moved along its corresponding axis 300a/b such that the head 296 of its syringe plunger 290a/b is aligned with the ram coupler 276, but with the axes 268 still in the offset configuration of Figure 7C, the syringe 286a/b may be translated within a plane that is orthogonal to the axis 268 along which the ram 274 moves. This establishes a coupled engagement between the ram coupler 276 and the syringe plunger coupler 296 in the above-noted manner.

The power injectors 210, 240 of Figures 6 and 7A-C each may be used for any appropriate application, including without limitation for medical imaging applications where fluid is injected into a subject (e.g., a patient) and/or any appropriate medical diagnostic and/or therapeutic application (e.g., injection of chemotherapy, pain management, etc.). Representative medical imaging applications for the power injectors 210,240 include without limitation computed tomography or CT imaging, magnetic resonance imaging or MRI, single photon emission computed tomography or SPECT imaging, positron emission tomography or PET imaging, X-ray imaging, angiographic imaging, optical imaging, and ultrasound imaging. The power injectors 210, 240 each could be used alone or in combination with one or more other components. The power injectors 210, 240 each may be operatively interconnected with one or more components, for instance so that information may be conveyed between the power injector 210, 240 and one or more other components (e.g., scan delay information, injection start signal, injection rate).

Any number of syringes may be utilized by each of the power injectors 210, 240, including without limitation single-head configurations (for a single syringe) and dual-head configurations (for two syringes). In the case of a multiple syringe configuration, each power injector 210, 240 may discharge fluid from the various syringes in any appropriate manner and according to any timing sequence (e.g., sequential discharges from two or more syringes, simultaneous discharges from two or more syringes, or any combination thereof). Multiple syringes may discharge into a common conduit (e.g., for provision to a single injection site), or one syringe may discharge into one conduit (e.g., for provision to one injection site), while another syringe may discharge into a different conduit (e.g., for provision to a different injection site). Each such syringe utilized by each of the power injectors 210,240 may include any appropriate fluid (e.g., a medical fluid), for instance contrast media, therapeutic fluid, a radiopharmaceutical, saline, and any combination thereof. Each such syringe utilized by each of the power injectors 210, 240 may be installed in any appropriate manner (e.g., rear-loading configurations may be utilized; front-loading configurations may be utilized; side-loading configurations may be utilized).

### EXAMPLES

Various polypropylene polymer products and clarifies were used to make polymer test compositions for testing as syringe barrels. Clarifier was added to some of the polymer products. Some of the polymer products may have also contained minor amounts of additives included by the manufacturer, which may have included some clarifier already added by the manufacturer. Some representative polypropylene products tested are identified in Table 1. Table 2 summarizes representative clarifiers tested through addition to various polypropylene products.

**Table 1**

| Polypropylene Polymer Product | Metallocene Polypropylene? | Reported Melt Flow Rate (230°C/2.16kg) | Reported Test Method |
|---|---|---|---|
| Bormed™ HD850MO, Borealis AG homopolymer | No | 8g/10min | ISO 1133 |
| ACHEIVE™ 1605, ExxonMobil Chemical Exxpol™ Metallocene Grade homopolymer | Yes | 32g/10min | ASTM D 1238 |
| ACHEIVE™ 3854, ExxonMobil Chemical Metallocene Grade homopolymer | Yes | 24 g/10min | ASTM D 1238 |
| P5C5N-062, Flint Hills Resources random copolymer | No | 20 g/10min | ASTM D 1238 |
| Pro-fax 6301, Lyondell Basell homopolymer | No | 12.0 g/10min | ASTM D 1238 |
| Metocene HM560R, Lyondell Basell homopolymer | Yes | 25 g/10min | ASTM D 1238 |
| Purell HP570M, Lyondell Basell homopolymer | No | 7.5 g/10min | ISO 1133 |
| Purell X50109, Lyondell Basell homopolymer | No | 60 g/10min | ASTM D 1238 |
| PP D-115-A, Sunoco Chemicals homopolymer | No | 11.0 g/10min | ASTM D 1238 |
| TR3350C, Sunoco Chemicals random copolymer | No | 35 g/10min | ASTM D 1238 |
| Polypropylene 3622, Total Petrochemicals homopolymer | No | 12 g/10min | ASTM D 1238 Condition "L" |
| Polypropylene 3825, Total Petrochemicals homopolymer | No | 30 g/10min | ASTM D 1238 Condition |
| Polypropylene 8573, Total Petrochemicals random copolymer | No | 6.8g/10mtn | ASTM D 1238 |
| Polypropylene 7824MR, Total Petrochemicals random copolymer | No | 27g/10min | ASTM D 1238 Condition "L" |
| Polypropylene M3766, Total Petrochemicals Metallocene Isotactic Propylene Polymer | Yes | 23g/10min | ASTM D 1238 |

**Table 2**

| Clarifiers | | |
|---|---|---|
| Amfine Chemical Corporation | NA-21 | Organophosphate |
| Milliken | Millad® NX8000 | Sorbital Acetal (4^{th} Generation) |
| Rika, New Japan Chemical Co., Ltd | Rikaclear® PC-1 | * |
| Ciba | IRGACLEAR® XT-386 | Trisamides |
| Milliken | NX-20 | Sorbital Acetal (4^{[h} Generation) |
| Milliken | Hyperform® HPN-20E | ** |

| | | |
|---|---|---|
| *Reported to contain (N,NI,NII-tris[2-methylcyclohexyl]-1,2,3-propaneticarbooxamide) **Reported to be a blend containing 66.67% cyclohexanedicarboxylic acid, calcium salt (1:1) and 33.33% zinc stearate. | | |

Test syringes were made using syringe barrels made from various test polymer compositions. Table 3 shows the composition of a number of test polymer compositions and the amount of clarifier added relative to the weight of the polymer product for those compositions to which clarifier is added. The starting polymer products and added clarifier are identified by manufacturer and product name. The polypropylene polymer in some of the polymer products was a polypropylene homopolymer, while in other polymer products, the polypropylene polymer was a polypropylene copolymer containing a small amount of ethylene repeating units.

Syringe barrels for the test syringes were made from the test polymer compositions by injection molding. The syringe barrels vary somewhat, but are generally about 20 cm long by 4 cm in diameter with an internal volume of about 125 mL. The syringe barrels were subjected to a variety of tests for evaluation. Not all tests were performed for all test polymer compositions or all test syringe barrels.

Burst Testing: A destructive burst test was performed in which a syringe assembly including a test syringe barrel and complete with piston (plunger) and piston backing plate, was hydraulically over-pressurized to failure by burst of the fluid-containment wall due to internal pressure within the syringe barrel. The syringe assembly outlet is restricted by a small bore needle and the piston backing plate was attached to a linear actuator equipped with appropriate strain gauge instrumentation. The linear actuator is advanced at a pre-determined rate until catastrophic failure of the syringe barrel occurred. The maximum pressure achieved by the syringe assembly was then calculated and recorded.

Impact Testing: A destructive impact test was performed in which the syringe assembly was retained in a cradle with TUP impact occurring mid-barrel. Instrumentation processes the signal and a trained operator interpreted the results. The maximum absorbed energy was then recorded. Some of the test syringe barrels withstood the impact insults without damage.

Extractability Testing: The syringe assemblies were tested for extractability of components from the fluid-containment walls of the syringe barrel. The test procedure was as specified in the Japanese pharmacopeia. UV absorbance was determined in a first range of UV wavelengths from 220 to 240 nanometers and a second range of UV wavelengths of 241-350 nanometers. A higher absorbance indicates a higher level of extractability of components from the wall of the syringe barrel. For some syringes, a visible observation was sufficient to identify a significant extractability problem with a particular test polymer composition. For some tests information was obtained only at a single wavelength, while for some other tests multiple measurements were made, as shown in Table 4.

Transparency Testing: The transparency of the fluid containment wall of the syringe barrel was measured according to the procedure provided in the Japanese pharmacopeia.

Representative results for the test compositions shown in Table 3 are summarized in Table 4. Tests that used a combination of metallocene polypropylene and organophosphate clarifier (test nos. 3, 5, 13 and 30) generally provided a combination of transparency of close to or above 50%, low extractability as indicated by a UV absorbance at 223-240 nm of no more than 0.08 and UV absorbance at 241-350 nm of no more than 0.05, burst pressure of at least 370 psi (2.55 MPa) and impact resistance of at least 0.2 foot pounds-force (0.0277 kg-force meter). Test compositions with Metallocene polypropylene and a non-organophosphate clarifier (test nos. 4 and 14) exhibited significantly higher levels of extractables. Of the test compositions not containing metallocene polypropylene, only two test compositions (test nos. 8 and 10) show reasonable results, although with noticeably higher levels of extractables than the noted tests with metallocene polypropylene and oganophosphate clarifier.

**Table 3**

| Test No. | Polymer Manufacturer and Product | Metallocene Polypropylene? | Homopolymer or Copolymer | Added Clarifier | Clarifier Type | Clarifier Amount wt % |
|---|---|---|---|---|---|---|
| 1 | Borealis Bormed HD850MO | No | Homopolymer | None | - | - |
| 2 | Exxon Mobil Achieve 1605 | Yes | Homopolymer | None | - | - |
| 3 | Exxon Mobil Achieve 1605 | Yes | Homopolymer | NA-21 | Organophosphate | 0.17 |
| 4 | Exxon Mobil Achieve 1605 | Yes | Homopolymer | NX8000 | Sorbital Acetal (4th generation) | 0.40 |
| 5 | Exxon Mobil Achieve 3854 | Yes | Homopolymer | NA-21 | Organophosphate | 0.17 |
| 6 | FHR P5C5N-062 | No | Copolymer | None | - | - |
| 7 | Lyondell Basell 6301 | No | Homopolymer | NA-21 | Organophosphate | 0.17 |
| 8 | Lyondell Basell 6301 | No | Homopolymer | NX8000 (18Melt) | Sorbital Acetal (4th generation) | 0.40 |
| 9 | Lyondell Basell 6301 | No | Homopolymer | NX8000 (22Melt) | Sorbital Acetal (4th generation) | 0.40 |
| 10 | Lyondell Basell 6301 | No | Homopolymer | Rikaclear PC-1 | Note 1 | 0.2 |
| 11 | Lyondell Basell 6301 | No | Homopolymer | XT-386 | Trisamides | 0.018 |
| 12 | Lyondell Basell Metocene HM560R | Yes | Homopolymer | None | - | - |
| 13 | Lyondell Basell Metocene HM560R | Yes | Homopolymer | NA-21 | Organophosphate | 0.17 |
| 14 | Lyondell Basell Metocene HM560R | Yes | Homopolymer | NX-20 | Sorbital Acetal (4th generation) | 0.40 |
| 15 | Lyondell Bassell HP570M | No | Homopolymer | None | - | - |
| 16 | Lyondell Bassell X50109 | No | Homopolymer | None | - | - |
| 17 | Lyondell Bassell X50109 | No | Homopolymer | NX-20 | Sorbital Acetal (4th generation) | 0.40 |
| 18 | Sunoco D115A | No | Homopolymer | None | - | - |
| 19 | Sunoco D115A | No | Homopolymer | HPN-20E | Note 2 | |
| 20 | Sunoco D115A | No | Homopolymer | NX-20 | Sorbital Acetal (4th generation) | 0.40 |
| 21 | Sunoco TR3350C | No | Copolymer | None | - | - |
| 22 | Total 3622 | No | Homopolymer | None | - | - |
| 23 | Total 3622 | No | Homopolymer | NA-21 | Organophosphate | 0.17 |
| 24 | Total 3622 | No | Homopolymer | NX8000 | Sorbital Acetal (4th generation) | 0.40 |
| 25 | Total 3622 | No | Homopolymer | Rikaclear PC-1 | Note 1 | 0.20 |
| 26 | Total 3622 | No | Homopolymer | XT-386 | Trisamides | 0.018 |
| 27 | Total 3825 | No | Homopolymer | None | - | - |
| 28 | Total 8573 | No | Copolymer | None | - | - |
| 29 | Total 7824WR | No | Copolymer | None | - | - |
| 30 | Total M3766 | Yes | Homopolymer | NA-21 | Organophosphate | 0.17 |

| | | | | | | |
|---|---|---|---|---|---|---|
| Note 1. Reported to contain (N,NI,NII-tris[2-methylcyclohexyl]-1,2,3-propaneticarbooxamide) Note 2. **Reported to be a blend containing 66.67% cyclohexanedicarboxylic acid, calcium salt (1:1) and 33.33% zinc stearate. | | | | | | |

**Table 4**

| **Test No.** | **Burst Mean psi** | **Impact Mean ft lb-force** | **UV 220-240 Absorbance** | **UV 241-350 Absorbance** | **Transparency %** | **Comments** |
|---|---|---|---|---|---|---|
| 1 | 435.88 | 135.32 | 0.083 @ 220 | 0.017 @ 241 | 20.67 | |
| 2 | 409.88 | Did Not Break | - | - | 32.77 | |
| 3 | 424.81 | 0.48 | 0.024 | 0.01 | 49.45 | |
| 4 | 428.63 | 0.42 | 0.245/0.112 | 0.145/0.071 | 64.05 | |
| 5 | 411.00 | 1.68 | 0.050 | 0.02 | 52.54 | |
| 6 | 388.94 | - | - | - | - | Visible Problem With Extractables |
| 7 | 415.56 | 0.33 | 0.068 | 0.034 | 39.41 | |
| 8 | 406.56 | 0.27 | 0.078 | 0.05 | 62.58 | |
| 9 | 405.63 | 0.23 | 0.186 | 0.14 | 55.43 | |
| 10 | 415.52 | 0.44 | 0.06 | 0.05 | 50.78 | |
| 11 | 431.25 | 0.45 | - | - | 46.07 | |
| 12 | 390.00 | Did Not Break | - | - | 34.08 | |
| 13 | 396.02 | 0.39 | 0.07 | 0.01 | 59.38 | |
| 14 | 413.69 | 0.10 | - | - | 70.98 | Visible Problem With Extractables |
| 15 | 397.30 | 0.17 | 0.047/0.047 | 0.027/0.022 | 29.55 | |
| 16 | 426.40 | 3.35 | 0.331 @ 220 | 0.147 @ 241 | 56.97 | |
| 17 | - | - | - | - | - | Not Processable/ Delaminated |
| 18 | 420.94 | 0.60 | - | - | 12.71 | Visible Problem With Extractables |
| 19 | - | - | - | - | - | |
| 20 | 410.00 | 0.11 | 0.101 | 0.056 | 62.55 | |
| 21 | 372.31 | 137.86 | - | - | - | Visible Problem With Extractables |
| 22 | - | - | 0.049/0.053 | 0.016/0.017 | - | |
| 23 | 418.70 | 0.08 | 0.051 | 0.02 | 32.42 | |
| 24 | 444.09 | 0.05 | 0.091/0.081 | 0.061/0.051 | 49.38 | |
| 25 | 423.32 | 0.06 | 0.06 | 0.03 | 39.22 | |
| 26 | 422.50 | 1.47 | 0.070 | 0.051 | 30.02 | |
| 27 | - | - | - | - | - | Visible Problem With Extractables |
| 28 | - | - | - | - | - | Visible Problem With Extractables |
| 29 | 299.13 | Did Not Break | - | - | - | Excessive Flexibility |
| 30 | 416.45 | 0.20 | 0.04 | 0.01 | 52.87 | |

The foregoing description of the present invention has been presented for purposes of illustration and description. Furthermore, the description is not intended to limit the invention to the form disclosed herein. Consequently, variations and modifications commensurate with the above teachings, and skill and knowledge of the relevant art, are within the scope of the present invention. The embodiments described hereinabove are further intended to explain best modes known of practicing the invention and to enable others skilled in the art to utilize the invention in such, or other embodiments and with various modifications required by the particular application(s) or use(s) of the present invention.

## Claims

1. A syringe product (40), comprising:
a syringe barrel (42) comprising a fluid-containment wall (48) and an internal volume (52) defined at least in part by the wall;
a syringe plunger (50) movably disposed within the internal volume and movable within the internal volume to accomplish at least one of creating fluid suction within at least a portion of the internal volume and creating fluid pressure within at least a portion of the internal volume;
the wall of the syringe barrel consisting essentially of a polymer composition comprising a metallocene polypropylene polymer and an organophosphate clarifier;
**characterised in that**
the polymer composition comprises at least 98 weight percent of the metallocene polypropylene polymer; and
the metallocene polypropylene polymer is polypropylene homopolymer.

2. A syringe product according to Claim 1, wherein the polymer composition comprises at least 99 weight percent of the metallocene polypropylene polymer and from 0.1 weight percent to 0.25 weight percent of the organophosphate clarifier.

3. A syringe product according to either one of Claim 1 or Claim 2, wherein the wall (48) has a transparency of at least 48%.

4. A syringe product according to any one of Claims 1-3, wherein the syringe barrel (42) has a low extractability indicated by extraction test absorbance for an ultraviolet wavelength spectrum of 220 to 240 nanometers of not greater than 0.08 and absorbance for an ultraviolet wavelength spectrum of 241 to 350 nanometers of not greater than 0.05, determined as specified in the Japanese pharmacopeia.

5. A syringe product according to any one of Claims 1-4, wherein the syringe barrel (42) is made substantially entirely of the polymer composition.

6. A syringe product according to any one of Claims 1-5, wherein the wall (48) is tubular and the internal volume is cylindrical.

7. A syringe product according to any one of Claims 1-6, wherein the syringe product (40) is a prefilled syringe comprising a volume of an injectable medical formulation sealed within the internal volume.

8. A syringe product according to Claim 7, wherein the syringe barrel (42) comprises a fluid exit port opposite the plunger with the injectable medical formulation disposed between the plunger and the fluid exit port, wherein the fluid exit port is sealed to prevent the injectable medical formulation from exiting through the fluid exit port.

9. A syringe product according to Claim 8, wherein the fluid exit port is not in fluid communication with a hypodermic needle.

10. A syringe product according to any one of Claims 7-9, wherein the injectable medical formulation comprises a member selected from the group consisting of x-ray contrast media, magnetic resonance contrast media, ultrasound imaging agents, optical imaging agents, saline, heparin and analgesics.

11. A syringe product according to any one of Claims 7-9, wherein the injectable medical formulation comprises x-ray contrast media.

12. A syringe product according to any one of Claims 7-9, wherein the injectable medical formulation comprises magnetic resonance contrast media.

13. A syringe product according to any one of Claims 7-9, wherein the injectable medical formulation comprises ultrasound imaging agent.

14. A syringe product according to any one of Claims 7-9, wherein the injectable medical formulation comprises optical imaging agent.

15. A method for preparing a filled syringe, comprising introducing a volume of an injectable medical formulation into the internal volume of the syringe product according to any one of Claims 1-6;
the injectable medical formulation comprising x-ray contrast media, magnetic resonance contrast media, ultrasound imaging agent or optical imaging agent.

16. A method according to Claim 15, comprising after the introducing, sealing the volume of injectable medical formulation in the internal volume.

17. Use of a polymer composition comprising at least 98 weight percent of a metallocene polypropylene homopolymer and an organophosphate clarifier for making a fluid-containment wall of a syringe barrel (42).

## Patentansprüche

1. Spritzenprodukt (40) mit:
einem Spritzengefäß (42), das eine fluidumhüllende Wand (48) und ein zumindest teilweise durch die Wand festgelegtes Innenvolumen (52) umfasst;
einem Spritzenkolben (50), der bewegbar im Innenvolumen (52) angeordnet ist und im Innenvolumen bewegbar ist, um zumindest entweder einen Fluidunterdruck in zumindest einem Abschnitt des Innenvolumens oder einen Fluidüberdruck in zumindest einem Abschnitt des Innenvolumens zu erzeugen;
wobei die Wand des Spritzengefäßes im Wesentlichen aus einer Polymerzusammensetzung besteht, die ein Metallocen-Propylen-Polymer und ein Organophosphat-Klärmittel umfasst,
**dadurch gekennzeichnet, dass**
die Polymerzusammensetzung mindestens 98 Gewichtsprozente des Metallocen-Propylen-Polymer enthält und
das Metallocen-Propylen-Polymer ein Polypropylen-Homopolymer ist.

2. Spritzenprodukt nach Anspruch 1, wobei die Polymerzusammensetzung mindestens 99 Gewichtsprozente des Metallocen-Propylen-Polymer und zwischen je einschließlich 0,1 Gewichtsprozent und 0,25 Gewichtsprozent des Organophosphat-Klärmittels enthält.

3. Spritzenprodukt nach Anspruch 1 oder Anspruch 2, wobei die Wand (48) eine Transparenz von mindestens 48% aufweist.

4. Spritzenprodukt nach einem der Ansprüche 1 bis 3, wobei das Spritzengefäß (42) eine geringe Extrahierbarkeit aufweist, die wie in der japanischen Pharmaverordnung festgelegt durch eine Absorption im Extrahierbarkeitstest für ein Ultraviolettwellenlängenspektrum zwischen 220 nm und 240 nm von nicht mehr als 0,08 und eine Absorption für ein Ultraviolettwellenlängenspektrum zwischen 241 und 350 nm von nicht mehr als 0,05 bestimmt ist.

5. Spritzenprodukt nach einem der Ansprüche 1 bis 4, wobei das Spritzengefäß (42) im Wesentlichen vollständig aus der Polymerzusammensetzung hergestellt ist.

6. Spritzenprodukt nach einem der Ansprüche 1 bis 5, wobei die Wand (48) röhrenförmig und das Innenvolumen zylindrisch ist.

7. Spritzenprodukt nach einem der Ansprüche 1 bis 6, wobei das Spritzenprodukt (40) eine vorbefüllte Spritze mit einem Volumen einer einspritzbaren medizinischen Rezeptur ist, die in dem Innenvolumen eingesiegelt ist.

8. Spritzenprodukt nach Anspruch 7, wobei das Spritzengefäß (42) einen Fluidausgangsanschluss gegenüber dem Kolben aufweist, wobei die einspritzbare medizinische Rezeptur zwischen dem Kolben und dem Fluidausgangsanschluss angeordnet ist, wobei der Fluidausgangsanschluss versiegelt ist, um zu verhindern, dass die einspritzbare medizinische Rezeptur durch den Fluidausgangsanschluss austritt.

9. Spritzenprodukt nach Anspruch 8, wobei der Fluidausgangsanschluss nicht in Fluidverbindung mit einer subkutanen Nadel steht.

10. Spritzenprodukt nach einem der Ansprüche 7 bis 9, wobei die einspritzbare medizinische Rezeptur etwas umfasst, das aus der Gruppe ausgewählt ist, die aus Röntgenkontrastmittel, Magnetresonanzkontrastmittel, Ultraschallbildgebungsmittel, optisches Bildgebungsmittel, Salzlösung, Heparin und Analgetika besteht.

11. Spritzenprodukt nach einem der Ansprüche 7 bis 9, wobei die einspritzbare medizinische Rezeptur Röntgenkontrastmittel umfasst.

12. Spritzenprodukt nach einem der Ansprüche 7 bis 9, wobei die einspritzbare medizinische Rezeptur Magnetresonanzkontrastmittel umfasst.

13. Spritzenprodukt nach einem der Ansprüche 7 bis 9, wobei die einspritzbare medizinische Rezeptur Ultraschallbildgebungsmittel umfasst.

14. Spritzenprodukt nach einem der Ansprüche 7 bis 9, wobei die einspritzbare medizinische Rezeptur optische Bildgebungsmittel umfasst.

15. Verfahren zum Vorbereiten einer gefüllten Spritze mit Einführen eines Volumen einer einspritzbaren medizinischen Rezeptur in das Innenvolumen des Spritzenprodukts nach einem der Ansprüche 1 bis 6;
wobei die einspritzbare medizinische Rezeptur Röntgenkontrastmittel, Magnetresonanzkontrastmittel, Ultraschallbildgebungsmittel oder optisches Bildgebungsmittel umfasst.

16. Verfahren nach Anspruch 15, mit Versiegeln des Volumens der einspritzbaren medizinischen Rezeptur in dem Innenvolumen nach dem Einführen.

17. Verwenden einer Polymerzusammensetzung, die mindestens 98 Gewichtsprozent eines Metallocen-Polypropylen-Homopolymers und ein Organophosphat-Klärmittel umfasst, zum Herstellen einer fluidumhüllenden Wand eines Spritzengefäßes (42).

## Revendications

1. Produit seringue (40), comprenant :
un corps de seringue (42) comprenant une paroi de retenue de fluide (48) et un volume interne (52) défini au moins en partie par la paroi ;
un piston de seringue (50) disposé de façon mobile dans le volume interne et mobile dans le volume interne de façon à effectuer au moins une action parmi la création d'une aspiration du fluide dans au moins une partie du volume interne et la création d'une pression de fluide dans au moins une partie du volume interne ;
la paroi du corps de seringue consistant essentiellement en une composition de polymère comprenant un polymère de polypropylène métallocène et un agent de clarification organophosphate ;
**caractérisé en ce que**
la composition de polymère comprend au moins 98 % en poids du polymère de polypropylène métallocène et
le polymère de polypropylène métallocène est un homopolymère de polypropylène.

2. Produit seringue selon la revendication 1, dans lequel la composition de polymère comprend au moins 99 % en poids du polymère de polypropylène métallocène et de 0,1 % en poids à 0,25 % en poids de l'agent de clarification organophosphate.

3. Produit seringue selon l'une quelconque des revendications 1 ou 2, dans lequel la paroi (48) a une transparence d'au moins 48 %.

4. Produit seringue selon l'une quelconque des revendications 1 à 3, dans lequel le corps de seringue (42) a une faible extractabilité indiquée par le test d'extraction par l'absorbance d'un spectre de longueur d'ondes ultraviolettes de 220 à 240 nanomètres ne dépassant pas 0,08 et l'absorbance d'un spectre de longueur d'ondes ultraviolettes de 241 à 350 nanomètres ne dépassant pas 0,05, déterminée comme le préconise la Pharmacopée japonaise.

5. Produit seringue selon l'une quelconque des revendications 1 à 4, dans lequel le corps de seringue (42) est constitué sensiblement entièrement de la composition de polymère.

6. Produit seringue selon l'une quelconque des revendications 1 à 5, dans lequel la paroi (48) est tubulaire et le volume interne est cylindrique.

7. Produit seringue selon l'une quelconque des revendications 1 à 6, le produit seringue (40) étant une seringue pré-remplie comprenant un volume d'une formulation médicale injectable scellée dans le volume interne.

8. Produit seringue selon la revendication 7, dans lequel le corps de seringue (42) comprend un orifice de sortie de fluide opposé au piston, la formulation médicale injectable étant disposée entre le piston et l'orifice de sortie de fluide, dans lequel l'orifice de sortie de fluide est scellé pour empêcher la formulation médicale injectable de sortir par l'orifice de sortie de fluide.

9. Produit seringue selon la revendication 8, dans lequel l'orifice de sortie de fluide n'est pas en communication de fluide avec une aiguille hypodermique.

10. Produit seringue selon l'une quelconque des revendications 7 à 9, dans lequel la formulation médicale injectable comprend un élément choisi dans le groupe comprenant des milieux de contraste radiographique, des milieux de contraste à résonance magnétique, des agents d'imagerie par ultrasons, des agents d'imagerie optique, une solution physiologique, l'héparine et des analgésiques.

11. Produit seringue selon l'une quelconque des revendications 7 à 9, dans lequel la formulation médicale injectable comprend des milieux de contraste radiographique.

12. Produit seringue selon l'une quelconque des revendications 7 à 9, dans lequel la formulation médicale injectable comprend des milieux de contraste à résonance magnétique.

13. Produit seringue selon l'une quelconque des revendications 7 à 9, dans lequel la formulation médicale injectable comprend un agent d'imagerie par ultrasons.

14. Produit seringue selon l'une quelconque des revendications 7 à 9, dans lequel la formulation médicale injectable comprend un agent d'imagerie optique.

15. Procédé de préparation d'une seringue remplie, comprenant l'introduction d'un volume d'une formulation médicale injectable dans le volume interne du produit de seringue selon l'une quelconque des revendications 1 à 6 ;
la formulation médicale injectable comprenant des milieux de contraste radiographique, des milieux de contraste à résonance magnétique, des agents d'imagerie par ultrasons ou des agents d'imagerie optique.

16. Procédé selon la revendication 15, comprenant après l'introduction, le scellement du volume de la formulation médicale injectable dans le volume interne.

17. Utilisation d'une composition polymère comprenant au moins 98 % en poids d'un homopolymère de polypropylène métallocène et un agent de clarification organophosphate pour produire une paroi de retenue de fluide d'un corps de seringue (42).
